# EUROPEAN PATENT APPLICATION

(11) **EP 4 000 566 A1**
(43) Date of publication of application: **25.05.2022**
(21) Application number: 20841276.7
(22) Date of filing: 16.07.2020
(51) Int. Cl.: A61F 2/82, A61M 1/36

(54) **VASCULAR CORRECTIVE DEVICE AND METHOD FOR SUPPORTING ANASTOMOTIC SITE**

(30) Priority: 17.07.2019 JP 2019131989
(71) Applicant: Tohoku University, Sendai-shi, Miyagi 980-8577 (JP)
(72) Inventor: KAJIYAMA Ai, Sendai-shi, Miyagi 980-8577 (JP); KINOSHITA Tomo, Sendai-shi, Miyagi 980-8577 (JP); TSUJIMOTO Yuiko, Sendai-shi, Miyagi 980-8577 (JP); YOSHIDA Shinya, Sendai-shi, Miyagi 980-8577 (JP); TAKASE Kei, Sendai-shi, Miyagi 980-8577 (JP); IZUMI Shinichi, Sendai-shi, Miyagi 980-8577 (JP); NAGATOMI Ryoichi, Sendai-shi, Miyagi 980-8577 (JP); HARA Yosuke, Sendai-shi, Miyagi 980-8577 (JP); TAKI Hirofumi, Sendai-shi, Miyagi 980-8577 (JP)
(74) Representative: Plougmann Vingtoft a/s
(86) International application number: PCT/JP2020/027623
(87) International publication number: WO 2021/010434

(57) **Abstract**

To provide a vascular corrective appliance and a supporting method for an anastomosis part capable of reducing or preventing narrowing at an anastomosis part between vessels and/or narrowing in the vicinity of the anastomosis part.

The vascular corrective appliance and the supporting method are configured to support a first vessel and a second vessel from their outer sides in such a manner that an angle formed between a blood flow direction of the first vessel and a blood flow direction of the second vessel at an anastomosis part where the first vessel is anastomosed in a branch pattern with the second vessel becomes an acute angle, and that the first vessel is curved on a downstream side from the anastomosis part to extend toward an upstream side of the second vessel from the anastomosis part.

## Description

### Field of the Invention

The present invention relates to a vascular corrective appliance and a supporting method for an anastomosis part using the vascular corrective appliance.

### Description of Related Art

For a patient with end-stage renal disease, a declined renal function is compensated for through hemodialysis performed by taking out blood through a vascular access formed inside a body, removing waste products or redundant water from the blood, and returning the purified blood into the body.

A shunt for hemodialysis mainly and conventionally used includes an arteriovenous fistula formed by anastomosing an artery and a vein of the patient himself or herself with each other, and an arteriovenous graft formed by anastomosing an artery and a vein of the patient, and a vascular graft with each other. Each of these shunts is formed in such a manner that, during anastomosing of a first vessel (access vessel) in a branch pattern with a second vessel, an angle formed between a blood flow direction of the first vessel and a blood flow direction of the second vessel at an anastomosis part becomes an obtuse angle. In these conventional shunts, blood is bent to an acute angle at the anastomosis part when entering the first vessel from the second vessel. This makes a blood flow collide with a vascular wall of the first vessel to cause a disturbed blood flow, thereby thickening the vascular wall. For this reason, the conventional shunts frequently cause malfunction due to narrowing inside the vessel, resulting in a problem of causing difficulty in implementation of hemodialysis. The narrowing occurs most frequently at the shunt anastomosis part or in the vicinity of the anastomosis part, which is a problem common between an arteriovenous fistula and an arteriovenous graft.

It is said that there are 300,000 dialysis patients at present in Japan and the above-described narrowing at the vein for dialysis shunt takes up a large share of dialysis-related troubles. The narrowing may be cured by a remedy of making anastomosis again, or there are several types of shunt construction devices such as a balloon catheter encouraging narrowing prevention. However, these produce only temporary effects and involve pains of patients or expensive cost for the remedy to cause serious problems. Hence, what is required is to construct and maintain a shunt allowing implementation of dialysis for a long time. While narrowing occurs at various locations such as a downstream area of a shunt vein and a site of puncture, the narrowing occurs most frequently in the vicinity of an anastomosis part and it is said that this narrowing is likely to induce narrowing at a different site. As a result, preventing the narrowing in the vicinity of the anastomosis part is believed to be a preventive against shunt narrowing in most cases.

In a medical device developed to solve the above-described problem of vessel narrowing, a first vessel and a second vessel are anastomosed with each other into a predetermined shape and the shape is retained, thereby allowing reduction in malfunction or defect in a vessel (see Patent Literatures 1 to 4, for example). This medical device includes an external vascular support with at least one vascular support wall for housing an external vascular wall of a vessel, the vascular support includes a part housing the first vessel and a part housing the second vessel, and each of these parts is formed into a shape in such a manner as to provide vessel rounding to at least a partial area of an anastomosis part.

In the medical device disclosed in each of Patent Literatures 1 to 4, an angle formed between a blood flow direction of the first vessel and a blood flow direction of the second vessel at the anastomosis part is an obtuse angle. A simulation about a blood flow was conducted by setting this angle to an acute angle, and result thereof shows that this reduces a disturbed blood flow compared to the case of the obtuse angle (see Non-Patent Literature 1, for example). Other reports show that shunt narrowing (intimal thickening) is likely to occur at a position where wall shear stress exhibits an abnormal value (see Non-Patent Literature 2, for example), and that controlling wall shear stress in a shunt to a normal value is effective in preventing shunt narrowing (intimal thickening) (see Non-Patent Literature 3), for example.

A simulation about a blood flow was conducted by setting an angle formed at the anastomosis part between a blood flow direction of the first vessel and a blood flow direction of the second vessel to 45°, 90°, and 135°. Result thereof shows that the angle of 45° reduces wall shear stress. In the case of an actual patient, it is also reported that setting an angle formed between a blood flow direction of the first vessel and a blood flow direction of the second vessel to an acute angle reduces wall shear stress at the anastomosis part or in the vicinity of the anastomosis part and reduces a shunt narrowing ratio (see Non-Patent Literature 4, for example).

### Citation List

### Patent Literatures

Patent Literature 1: JP-B-6356065
Patent Literature 2: JP-B-6522828
Patent Literature 3: US-B-10004508
Patent Literature 4: WO 2014/020565

### Non-Patent Literatures

Non-Patent Literature 1: John Carroll, Ramon L Varcoe, Tracie Barber And Anne Simmons, "Reduction in anastomotic flow disturbance within a modified end-to-side arteriovenous fistula configuration: Results of a computational flow dynamic model", Nephrology, 2019, 24, pp. 245-251
Non-Patent Literature 2: LAN JIA, et al., "Effects of wall shear stress in venous neointimal hyperplasia of arteriovenous fistulae", Nephrology, 2015, 20, pp. 335-342
Non-Patent Literature 3: Timmy Lee and Sanjay Misra, "New Insights into Dialysis ascular Access: Molecular Targets in Arteriovenous Fistula and Arteriovenous Graft Failure and Their Potential to Improve Vascular Access Outcomes", Clin. J. Am. Soc. Nephrol., 2016, 11, pp. 1504-1512
Non-Patent Literature 4: Jinkee Lee, et al., "Assessing radiocephalic wrist arteriovenous fistulas of obtuse anastomosis part using computational fluid dynamics and clinical application", J Vase. Access, 2016, 17 (6), pp. 512-520

### Summary of the Invention

In the medical device disclosed in each of Patent Literatures 1 to 4, an angle formed between a blood flow direction of the first vessel and a blood flow direction of the second vessel at the anastomosis part is an obtuse angle, thereby bending a blood flow to an acute angle. This causes a problem that limitation is imposed on suppressing the occurrence of a disturbed blood flow and limitation is also imposed on preventing narrowing in a vessel. There is currently no device for providing support while an angle formed between a blood flow direction of the first vessel and a blood flow direction of the second vessel at the anastomosis part is an acute angle.

The present invention has been made focusing on those problems. It is an object of the present invention to provide a vascular corrective appliance and a supporting method for an anastomosis part capable of reducing or preventing narrowing at an anastomosis part between vessels and/or narrowing in the vicinity of the anastomosis part.

To attain the above object, the vascular corrective appliance according to the present invention is a vascular corrective appliance used in anastomosing a first vessel in a branch pattern with a second vessel, and configured to support the first vessel and the second vessel from their outer sides in such a manner that an angle formed between a blood flow direction of the first vessel and a blood flow direction of the second vessel at an anastomosis part becomes an acute angle, and that the first vessel is curved on a downstream side from the anastomosis part to extend toward an upstream side of the second vessel from the anastomosis part.

The vascular corrective appliance according to the present invention is applied preferably to a part where the first vessel is anastomosed in a branch pattern with the second vessel in such a manner that an angle formed between a blood flow direction of the first vessel and a blood flow direction of the second vessel becomes an acute angle. The vascular corrective appliance according to the present invention can support the first vessel and the second vessel from their outer sides in such a manner that an angle formed between a blood flow direction of the first vessel and a blood flow direction of the second vessel at the anastomosis part becomes an acute angle. It is also possible to support the first vessel and the second vessel in such a manner that the first vessel is curved on a downstream side from the anastomosis part to extend toward an upstream side of the second vessel from the anastomosis part. Thus, compared to a case where a blood flow is bent to an acute angle at the anastomosis part, the vascular corrective appliance according to the present invention reduces the occurrence of a disturbed blood flow and change in wall shear stress effectively to suppress thickening of a vascular wall, making it possible to reduce or prevent narrowing at an anastomosis part between vessels and/or narrowing in the vicinity of the anastomosis part. The disturbed blood flow is a disturbed flow of blood and means a disturbed flow compared to a normal blood flow caused by a factor such as sudden change in the geometric structure of a vessel or sudden change in a blood flow rate. The occurrence of the disturbed blood flow may cause dynamic influence on vascular endothelial cells, thereby causing sudden change in wall shear stress or causing an abnormal wall shear stress distribution to become a cause for narrowing, etc.

To correct a blood flow hydrodynamically, the vascular corrective appliance according to the present invention can support anastomosed vessels from their outer sides. The vascular corrective appliance according to the present invention can suppress or prevent unfavorable change by adjusting ways of flows inside the anastomosed vessels, for example. The unfavorable change may result from intimal hyperplasia, vessel constriction, and/or narrowing at a lumen, for example. The vascular corrective appliance according to the present invention can suppress or prevent such occurrences. The vascular corrective appliance according to the present invention is applicable to any patient in need of vascular anastomosis and is used for a patient in need of implementation of hemodialysis, for example.

In the present description, the term "vessel" means a tube through which blood is sent to each part of a body. For example, the vessel means a vein, an artery, or a vascular graft. The term "anastomosis part" means a joint part where the first vessel (vein, artery, or vascular graft) is anastomosed with the second vessel (vein, artery, or vascular graft) to allow a flow from inside of the first vessel into the second vessel and to allow a reverse flow. Suh anastomosis can be realized by means of suture or other means. The term "vascular graft" means an artificial tubular element used as a vessel and to indwell in a body. For example, this term means an element prepared using cloth, PTFE, a biomaterial, a synthetic polymer material, a biological polymer material, cells, or a combination of such materials.

Preferably, the vascular corrective appliance according the present invention is configured to support the first vessel and the second vessel in such a manner that the first vessel is curved with a radius of curvature from 0.1 to 300 mm in a length range of equal to or less than 10 cm from the anastomosis part toward a downstream side to extend toward an upstream side of the second vessel from the anastomosis part. In this case, narrowing at a vessel can be reduced or prevented more effectively. A range of the curvature of the first vessel may be the entire length range of equal to or less than 10 cm from the anastomosis part toward a downstream side, or may be a part of this range. The radius of curvature of the first vessel is preferably from 10 to 50 mm, particularly preferably, from 20 to 30 mm.

Preferably, the vascular corrective appliance according to the present invention is configured to form the shapes of the first vessel and the second vessel and retain the shapes in such a manner that the first vessel is rounded on a downstream side from the anastomosis part toward an upstream side of the second vessel from the anastomosis part, and that the rounding has a radius of curvature from 0.1 to 300 mm in a length range of equal to or less than 10 cm from the anastomosis part toward a downstream side. This can also reduce or prevent narrowing at a vessel more effectively. A range of the rounding of the first vessel with a radius of curvature from 0.1 to 300 mm may be the entire length range of equal to or less than 10 cm from the anastomosis part toward a downstream side, or may be a part of this range. The rounding of the first vessel is preferably from 10 to 50 mm, particularly preferably, from 20 to 30 mm.

In particular, the term "rounding" in the present description is rounding of a vessel at an anastomosis part and/or in the vicinity of the anastomosis part that avoids a sharp edge or corner at the anastomosis part and/or in the vicinity of the anastomosis part. Such rounding of a vessel is given particularly to anastomosis between vessels or anastomosis between a vessel and a vascular graft. The rounding is a partially curved shape, meaning that this is not straight. Specifically, regarding an arbitrary point on an outer side of a rounded vessel with a larger radius of curvature, assuming that the center of a circle giving a curvature to this point is defined as A, and a point on an outer side of the vessel with a smaller radius of curvature and a point on an outer side of the vessel with a larger radius of curvature existing on a straight line passing through the point A are defined as B and C respectively, a distance between A and C is greater than a distance between A and B. The rounding should not be confused with the contour of a vessel entirely or the contour of one section of the vessel but has a particular curvature along the length of the vessel. The rounding in the present description should not be confused with the shape of a transversal plane of a vascular wall.

In the vascular corrective appliance according to the present invention, an angle formed between a blood flow direction of the first vessel and a blood flow direction of the second vessel at the anastomosis part is preferably equal to or greater than 20 degrees and less than 90, more preferably, from 20 to 60 degrees, particularly preferably, from 20 to 40 degrees. This makes it possible to reduce a disturbed blood flow and wall shear stress compared to a case where this angle is an obtuse angle. Thus, it becomes possible to reduce or prevent narrowing at an anastomosis part between vessels and/or narrowing in the vicinity of the anastomosis part more effectively.

The vascular corrective appliance according to the present invention may support the first vessel and the second vessel in any way as long as the support is given from their outer sides, and may have any configuration and shape. For example, the vascular corrective appliance according to the present invention may be configured to cover a predetermined range partially or entirely toward a downstream side of the first vessel from the anastomosis part, to cover a predetermined range partially or entirely toward an upstream side of the second vessel from the anastomosis part, and to cover a predetermined range partially or entirely toward a downstream side of the second vessel from the anastomosis part from the outer sides of the first vessel and the second vessel. The vascular corrective appliance according to the present invention may also be configured to cover a predetermined range partially or entirely toward a downstream side of the first vessel from the anastomosis part and to cover a predetermined range partially or entirely toward an upstream side of the second vessel from the anastomosis part from the outer sides of the first vessel and the second vessel. The vascular corrective appliance according to the present invention may also be configured to cover a predetermined range partially or entirely toward a downstream side of the first vessel from the anastomosis part and to cover a predetermined range partially or entirely toward a downstream side of the second vessel from the anastomosis part from the outer sides of the first vessel and the second vessel.

For example, the vascular corrective appliance according to the present invention may be provided in the first vessel on its outer side in the vicinity of the anastomosis part and in the second vessel on its outer side, may be provided in the second vessel on its outer side in the vicinity of the anastomosis part and in the first vessel on its outer side with a smaller radius of curvature, or may be provided in the second vessel on its outer side in the vicinity of the anastomosis part and in the first vessel on its outer side with a larger radius of curvature.

The vascular corrective appliance according to the present invention may comprise two divisions dividable and combinable along a plane including the center line of the first vessel and the center line of the second vessel, and the vascular corrective appliance may be configured to cover the first vessel and the second vessel by attaching and combining the divisions from opposite sides of each of the first vessel and the second vessel. The vascular corrective appliance according to the present invention may have a rod shape or a plate shape with a curvature. The vascular corrective appliance according to the present invention may be configured to be fixed to vessels in the vicinity of the anastomosis part by providing a C-shaped vessel gripper at each of parts of the vascular corrective appliance to be arranged on its outer side in the first vessel and in the second vessel in the vicinity of the anastomosis part on its outer side, and by causing the vessel grippers to grip corresponding ones of the first vessel and the second vessel.

Preferably, in the vascular corrective appliance according to the present invention, each of the first vessel and the second vessel is an artery, a vein, or a vascular graft. This allows application to an arteriovenous fistula or an arteriovenous graft. In particular, for a patient in need of implementation of hemodialysis, for example, the first vessel is preferably a vein and the second vessel is preferably an artery.

Preferably, the vascular corrective appliance according to the present invention is configured to be adjustable in terms of dimension and/or shape. In this case, during attachment to the first vessel and the second vessel, the vascular corrective appliance can be adjusted for each patient to an optimum dimension or shape. The vascular corrective appliance according to the present invention may be coated with a chemical for suppressing vascular endothelial growth. In this case, thickening of a vascular wall can be suppressed using the chemical, making it possible to reduce or prevent narrowing at a vessel more effectively.

The supporting method for the anastomosis part according to the present invention uses the vascular corrective appliance according to the present invention to support the first vessel and the second vessel anastomosed with each other.

By the use of the vascular corrective appliance according to the present invention, the supporting method for the anastomosis part according to the present invention can support the first vessel and the second vessel from their outer sides in such a manner that an angle formed between a blood flow direction of the first vessel and a blood flow direction of the second vessel at the anastomosis part becomes an acute angle. It is also possible to support the first vessel and the second vessel in such a manner that the first vessel is curved on a downstream side from the anastomosis part to extend toward an upstream side of the second vessel from the anastomosis part. Thus, compared to a case where a blood flow is bent to an acute angle at the anastomosis part, the supporting method for the anastomosis part according to the present invention reduces the occurrence of a disturbed blood flow and change in wall shear stress effectively to suppress thickening of a vascular wall, making it possible to reduce or prevent narrowing at an anastomosis part between vessels and/or narrowing in the vicinity of the anastomosis part.

The present invention can provide a vascular corrective appliance and a supporting method for an anastomosis part capable of reducing or preventing narrowing at an anastomosis part between vessels and/or narrowing in the vicinity of the anastomosis part.

### Brief Description of the Drawings

FIG. 1 is a perspective view illustrating a vascular corrective appliance according to an embodiment of the present invention.
FIG. 2 is a perspective view illustrating a first modification of the vascular corrective appliance according to the embodiment of the present invention.
FIG. 3 is a perspective view illustrating a second modification of the vascular corrective appliance according to the embodiment of the present invention.
FIG. 4 includes (a) a side view of a third modification and (b) a side view of a fourth modification, each illustrating the vascular corrective appliance and a supporting method for an anastomosis part according to the embodiment of the present invention.
FIG. 5 includes (a) a side view of a fifth modification and (b) a side view of a sixth modification, each illustrating the vascular corrective appliance and the supporting method for the anastomosis part according to the embodiment of the present invention.

### Detailed Description of the Invention

Embodiments of the present invention will be described on the basis of the drawings.

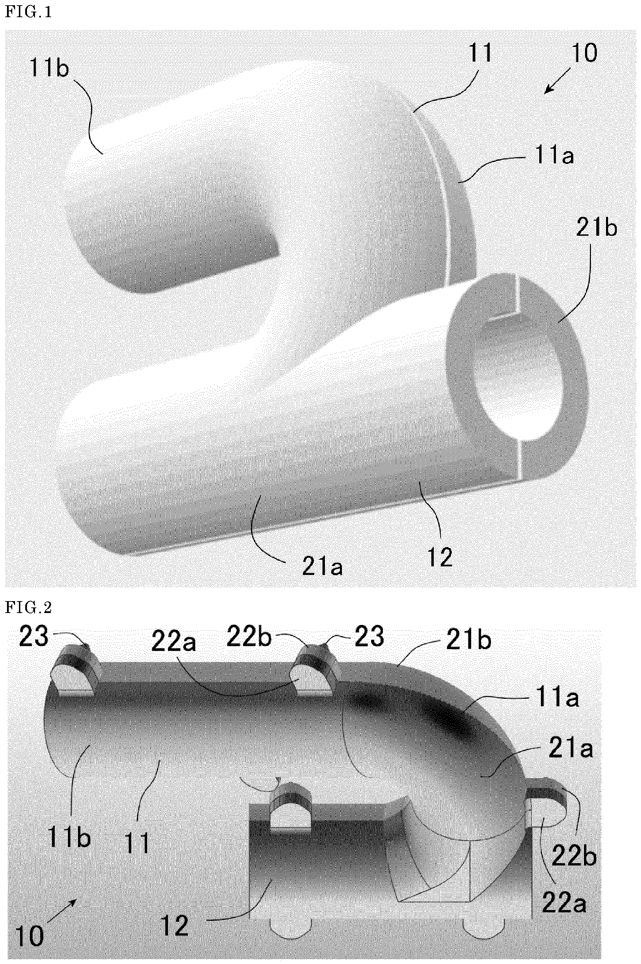

FIGS. 1 to 5 illustrate a vascular corrective appliance and a supporting method for an anastomosis part according to the embodiment of the present invention. The vascular corrective appliance and the supporting method for an anastomosis part according to the embodiment of the present invention are applied to a vascular access, for example, formed by anastomosing a first vessel in a branch pattern with a second vessel in such a manner that an angle formed between a blood flow direction of the first direction and a blood flow direction of the second vessel becomes an acute angle.

As shown in FIG. 1, a vascular corrective appliance 10 includes a first support 11 and a second support 12 provided integrally.

The first support 11 has an elongated tubular shape and forms an annular shape in a section vertical to a length direction. The first support 11 has one end portion 11a curved into an arc-like shape, and an opposite end portion 11b extending linearly. The one end portion 11a of the first support 11 forms an arc-like shape with a center angle of greater than 90 degrees. The second support 12 has an elongated tubular shape and forms an annular shape in a section vertical to a length direction having the same inner diameter and the same outer diameter as the first support 11. The second support 12 extends linearly.

In the vascular corrective appliance 10, the one end portion 11a of the first support 11 is connected to a central portion of the second support 12 in such a manner as to establish communication between the interior of the first support 11 and the interior of the second support 12. The vascular corrective appliance 10 is provided in such a manner that a direction in which the opposite end portion 11b of the first support 11 extends and a direction in which the second support 12 extends become parallel to each other, and that the center line of the first support 11 and the center line of the second support 12 are contained in the same plane. The vascular corrective appliance 10 is provided in such a manner that a connection between an inner section of the curve of the one end portion 11a of the first support 11 and the second support 12 has a smoothly and continuously extending shape on each of an outer side surface and on an inner side surface.

The vascular corrective appliance 10 is configured to support the first vessel and the second vessel from their outer sides. The vascular corrective appliance 10 is provided in such a manner that, while an anastomosis part between the first vessel and the second vessel is arranged at the connection between the first support 11 and the second support 12, the first vessel is housed in the first support 11 and the second vessel is housed in the second support 12. By doing so, the vascular corrective appliance 10 is configured to provide support in such a manner that an angle formed between a blood flow direction of the first vessel and a blood flow direction of the second vessel at the anastomosis part becomes an acute angle and that the first vessel is curved on a downstream side from the anastomosis part to extend toward an upstream side of the second vessel from the anastomosis part, and is configured to retain the resultant shapes of the first vessel and the second vessel.

In the specific example shown in FIG. 1, in the vascular corrective appliance 10, the one end portion 11a of the first support 11 is curved with a radius of curvature from 0.1 to 300 mm in a length range of equal to or less than 10 cm from a position of the connection to the second support 12 toward the opposite end portion 11b. By doing so, the vascular corrective appliance 10 is configured to support the first vessel and the second vessel in such a manner that the first vessel is curved with a radius of curvature from 0.1 to 300 mm in the length range of equal to or less than 10 cm from the anastomosis part toward a downstream side to extend toward an upstream side of the second vessel from the anastomosis part.

In the vascular corrective appliance 10, the one end portion 11a of the first support 11 may be rounded with a radius of curvature from 0.1 to 300 mm in the length range of equal to or less than 10 cm from the position of the connection to the second support 12 toward the opposite end portion 11b. By doing so, the vascular corrective appliance 10 is configured to form the shapes of the first vessel and the second vessel and retain the shapes in such a manner that the first vessel is rounded on a downstream side from the anastomosis part toward an upstream side of the second vessel from the anastomosis part, and that the rounding has a radius of curvature from 0.1 to 300 mm in the length range of equal to or less than 10 cm from the anastomosis part toward a downstream side.

In the specific example shown in FIG. 1, the vascular corrective appliance 10 is configured to be divided into two divisions 21a and 21b along a plane including the center line of the first support 11 and the center line of the second support 12. In this way, the vascular corrective appliance 10 includes the two divisions 21a and 21b dividable and combinable along a plane including the center line of the first vessel and the center line of the second vessel, and is configured to cover the first vessel and the second vessel by attaching and combining the divisions 21a and 21b from opposite sides of each of the first vessel and the second vessel.

In the specific example shown in FIG. 1, in the vascular corrective appliance 10, the respective inner diameters of the first support 11 and the second support 12 are determined to be slightly greater than the outer diameters of vessels to be housed in the first support 11 and the second support 12. The vascular corrective appliance 10 is configured to touch a vessel and configured to indwell in a body. Preferably, the vascular corrective appliance 10 is made of synthetic resin, metal, cells, or a material prepared by combining these materials, for example, and particularly preferably, is made of a biocompatible material as it is assumed to be implanted in a living body. If the biocompatible material mentioned herein is resin, it may be biodegradable polyester fluorine resin (such as polytetrafluoroethylene (PTFE) or polyurethane), polyetheretherketone (PEEK), for example. If the biocompatible material is metal, it may be titanium alloy, stainless steel, or cobalt alloy, for example. The vascular corrective appliance 10 may be made of a material having rigidity, semi-rigidity, or elasticity, or may be made of a flexible material.

Each of the first vessel and the second vessel is an artery, a vein, or a vascular graft, and the vascular corrective appliance 10 is applicable to an arteriovenous fistula and an arteriovenous graft. For a patient in need of implementation of hemodialysis, for example, the first vessel is a vein and the second vessel is an artery.

The supporting method for the anastomosis part according to the embodiment of the present invention is a method of supporting the anastomosed first vessel and second vessel using the vascular corrective appliance 10. The vascular corrective appliance 10 and the supporting method for the anastomosis part according to the embodiment of the present invention can support the first vessel and the second vessel from their outer sides in such a manner that an angle formed between a blood flow direction of the first vessel and a blood flow direction of the second vessel at the anastomosis part becomes an acute angle. The first vessel and the second vessel can also be supported in such a manner that the first vessel is curved on a downstream side from the anastomosis part to extend toward an upstream side of the second vessel from the anastomosis part. Thus, compared to a case where a blood flow is bent to an acute angle at the anastomosis part, the occurrence of a disturbed blood flow and change in wall shear stress are reduced effectively to suppress thickening of a vascular wall, making it possible to reduce or prevent narrowing at an anastomosis part between vessels and/or narrowing in the vicinity of the anastomosis part.

To correct a blood flow hydrodynamically, the vascular corrective appliance 10 and the supporting method for the anastomosis part according to the embodiment of the present invention can support anastomosed vessels from their outer sides. The vascular corrective appliance 10 and the supporting method for the anastomosis part according to the embodiment of the present invention can suppress or prevent unfavorable change by adjusting ways of flows inside the anastomosed vessels, for example. The unfavorable change may result from intimal hyperplasia, vessel constriction, and/or narrowing at a lumen. The vascular corrective appliance 10 and the supporting method for the anastomosis part according to the embodiment of the present invention can suppress or prevent such occurrences. For example, the vascular corrective appliance 10 and the supporting method for the anastomosis part according to the embodiment of the present invention can reduce or prevent malfunction in a vascular access for hemodialysis. The vascular corrective appliance 10 and the supporting method for the anastomosis part according to the embodiment of the present invention are applicable to any patient in need of anastomosis.

A simulation about a blood flow was conducted on the vascular corrective appliance 10 and the supporting method for the anastomosis part according to the embodiment of the present invention in the same way as in Non-Patent Literature 4. It is determined from result thereof that, if an angle formed between a blood flow direction of the first vessel and a blood flow direction of the second vessel at the anastomosis part is in a range from 20 to 90 degrees, a disturbed blood flow and wall shear stress are reduced compared to a case where this angle is an obtuse angle. It is further determined from result of the simulation that, with the radius of curvature of the first vessel on a downstream side from the anastomosis part from 10 to 50 mm and with an angle formed between a blood flow direction of the first vessel and a blood flow direction of the second vessel at the anastomosis part in a range from 20 to 90 degrees, wall shear stress is reduced monotonously as this angle becomes smaller.

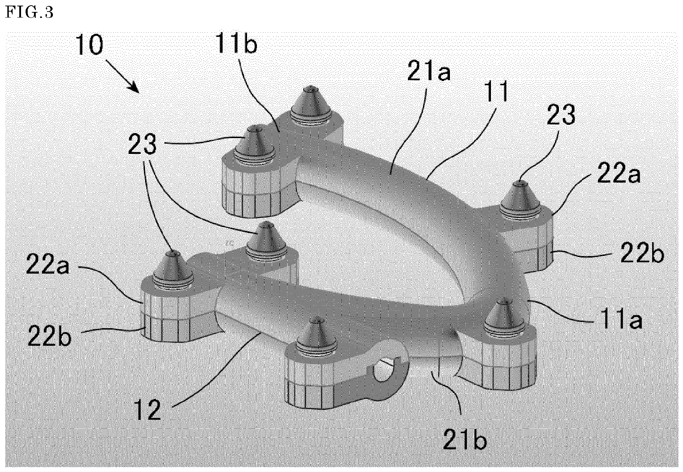

As shown in FIG. 2, the vascular corrective appliance 10 may be formed in such a manner that the opposite end portion 11b of the first support 11 extends longer than an end portion of the second support 12 on the same side. As shown in FIG. 3, in the vascular corrective appliance 10, the first support 11 and the second support 12 may be provided only on a downstream side from the anastomosis part. As shown in FIGS. 2 and 3, in the vascular corrective appliance 10, the division 21a may include protrusions 22a provided at a plurality of positions facing each other and the division 21b may include protrusions 22b provided at a plurality of positions facing each other. The protrusion 22a of the division 21a and the protrusion 22b of the division 21b may be fixed to each other using a fastening tool 23 such as a screw after the divisions 21a and 21b are combined in such a manner as to cover the first vessel and the second vessel. In the specific example shown in each of FIGS. 2 and 3, the one end portion 11a of the first support 11 is curved with a radius of curvature from 20 to 30 mm. An angle formed between a blood flow direction of the first vessel and a blood flow direction of the second vessel at the anastomosis part is 30 degrees.

The vascular corrective appliance 10 is not limited to the configurations and shapes shown in FIGS. 1 and 2 but may have any configuration and any shape and may be a porous member or a mesh member, for example, as long as such a configuration or shape allows shape forming of anastomosis and retention of the resultant shape and allows support of the first vessel and the second vessel from their outer sides. For example, the vascular corrective appliance 10 preferably includes at least one part for shape forming of anastomosis and retaining the resultant shape in a range including the first vessel contacting the anastomosis part, the anastomosis part, and the second vessel contacting the anastomosis part. The vascular corrective appliance 10 may also include at least one part for shape forming of the first vessel in the vicinity of the anastomosis part and retaining the resultant shape. The vascular corrective appliance 10 may also include a part for shape forming of anastomosis and retaining the resultant shape provided at each of a side with a smaller radius of curvature and a side with a larger radius of curvature of the curved one end portion 11a of the first vessel.

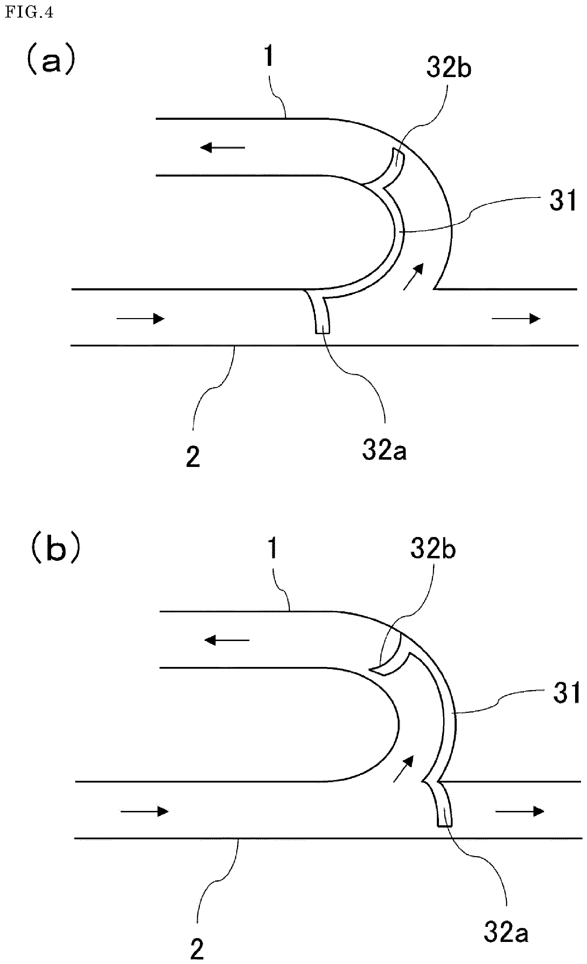

As shown in FIG. 4(a), for example, the vascular corrective appliance 10 may be provided in such a manner as to cover a predetermined range partially or entirely toward a downstream side of a first vessel 1 from an anastomosis part and to cover a predetermined range partially or entirely toward an upstream side of a second vessel 2 from the anastomosis part from the outer sides of the first vessel 1 and the second vessel 2. (Arrows in the drawing show blood flow directions. This further applies to the drawings referred to in the following.) The specific example shown in FIG. 4(a) includes a support plate 31 having a curvature and having an elongated plate-like shape, and C-shaped vessel grippers 32a and 32b in a pair provided at opposite ends of the support plate 31. Each of the vessel grippers 32a and 32b is attached at its central portion to the support plate 31 in such a manner as to be opened externally relative to the support plate 31. In this case, attachment may be formed in such a manner that the support plate 31 is arranged at an outer side surface of the first vessel 1 with a smaller radius of curvature, and that the vessel grippers 32a and 32b grip the second vessel 2 in the vicinity of the anastomosis part and the first vessel 1 respectively.

As shown in FIG. 4(b), the vascular corrective appliance 10 may be provided in such a manner as to cover a predetermined range partially or entirely toward a downstream side of a first vessel 1 from an anastomosis part and to cover a predetermined range partially or entirely toward a downstream side of a second vessel 2 from the anastomosis part from the outer sides of the first vessel 1 and the second vessel

2. The specific example shown in FIG. 4(b) includes a support plate 31 having a curvature and having an elongated plate-like shape, and C-shaped vessel grippers 32a and 32b in a pair provided at opposite ends of the support plate 31. One vessel gripper 32a is attached at its central portion to the support plate 31 in such a manner as to be opened externally relative to the support plate 31. The other vessel gripper 32b is attached at its central portion to the support plate 31 in such a manner as to be opened internally relative to the support plate 31. In this case, attachment may be formed in such a manner that the support plate 31 is arranged at an outer side surface of the first vessel 1 with a larger radius of curvature, the one vessel gripper 32a grips the second vessel 2 in the vicinity of the anastomosis part, and the other vessel gripper 32b grips the first vessel 1.

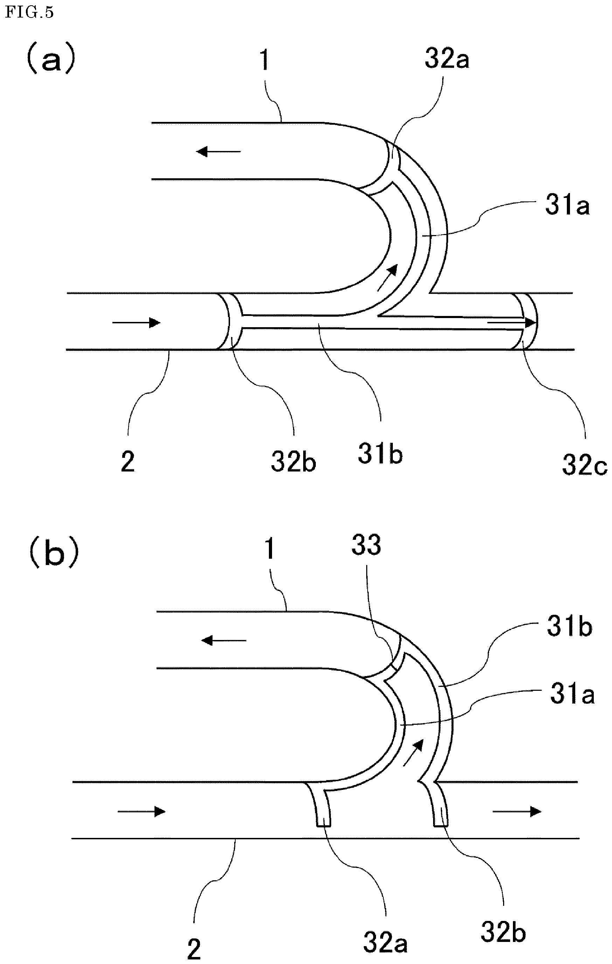

As shown in FIGS. 5(a) and (b), the vascular corrective appliance 10 may be provided in such a manner as to cover a predetermined range partially or entirely toward a downstream side of a first vessel 1 from an anastomosis part, to cover a predetermined range partially or entirely toward an upstream side of a second vessel 2 from the anastomosis part, and to cover a predetermined range partially or entirely toward a downstream side of the second vessel 2 from the anastomosis part from the outer sides of the first vessel 1 and the second vessel 2. The specific example shown in FIG. 5(a) includes a first support plate 31a having a planar shape that is an elongated arc-like shape, an elongated plate-like second support plate 31b, and three C-shaped vessel grippers 32a, 32b, and 32c provided at one end of the first support plate 31a and opposite ends of the second support plate 31b. An opposite end of the first support plate 31a is connected to a central portion of the second support plate 31b. The vessel grippers 32a, 32b, and 32c are attached at their central portions to corresponding ones of the support plates 31a and 31b in such a manner as to be opened toward the same side relative to the first support plate 31a and the second support plate 31b. In this case, attachment may be formed in such a manner that the first support plate 31a and the second support plate 31b are arranged at a side surface of the first vessel 1 and at a side surface of the second vessel 2 respectively, the vessel gripper 32a attached to the first support plate 31a grips the first vessel 1, and the two vessel grippers 32b and 32c attached to the second support plate 31b grip the second vessel 2.

The specific example shown in FIG. 5(b) includes a first support plate 31a and a second support plate 31b facing each other, substantially parallel to each other, and each having an elongated plate-like shape with a curvature, an annular vessel gripper 33 provided at one end of the first support plate 31a and one end of the second support plate 31b in such a manner as to connect the first support plate 31a and the second support plate 31b to each other, and a C-shaped vessel gripper 32a and a C-shaped vessel gripper 32b provided at an opposite end of the first support plate 31a and at an opposite end of the second support plate 31b respectively. One vessel gripper 32a is attached at its central portion to the first support plate 31a in such a manner as to be opened externally relative to the first support plate 31a. The other vessel gripper 32b is attached at its central portion of the support plate 31b in such a manner as to be opened externally relative to the second support plate 31b. In this case, attachment may be formed in such a manner that the first support plate 31a is arranged at an outer side surface of the first vessel 1 with a smaller radius of curvature, the second support plate 31b is arranged at an outer side surface of the first vessel 1 with a larger radius of curvature, the vessel gripper 33 grips the first vessel 1 inserted in the inner side of the vessel gripper 33, one vessel gripper 32a grips an upstream side of the second vessel 2 from the anastomosis part, and the other vessel griper 32b grips a downstream side of the second vessel 22 from the anastomosis part.

The vascular corrective appliance 10 may be configured to be adjustable in terms of dimension and/or shape. In this case, during attachment to the first vessel 1 and the second vessel 2, the vascular corrective appliance 10 can be adjusted for each patient to an optimum dimension or shape. The vascular corrective appliance 10 may be coated with a chemical for suppressing vascular endothelial growth. In this case, thickening of a vascular wall can be suppressed using the chemical, making it possible to reduce or prevent narrowing at a vessel more effectively.

### Industrial Applicability

The vascular corrective appliance and the supporting method for the anastomosis part according to the present invention are applied to a shunt such as an arteriovenous fistula or an arteriovenous graft, and are capable of suppressing narrowing occurring at an anastomosis part of the shunt or at a downstream area of a shunt vein, for example. By doing so, burden on dialysis patients is expected to be reduced considerably, for example.

### Reference Signs List

10: Vascular corrective appliance
11: First support
   11a: One end portion
   11b: Opposite end portion
12: Second support
21a, 21b: Division
22a, 22b: Protrusion
23: Fastening tool
   1: First vessel
   2: Second vessel
31: Support plate
31a: First support plate
31b: Second support plate
32a, 32b, 32c, 33: Vessel gripper

## Claims

1. A vascular corrective appliance used in anastomosing a first vessel in a branch pattern with a second vessel,
the vascular corrective appliance being configured to support the first vessel and the second vessel from their outer sides in such a manner that an angle formed between a blood flow direction of the first vessel and a blood flow direction of the second vessel at an anastomosis part becomes an acute angle, and that the first vessel is curved on a downstream side from the anastomosis part to extend toward an upstream side of the second vessel from the anastomosis part.

2. The vascular corrective appliance according to claim 1, configured to support the first vessel and the second vessel in such a manner that the first vessel is curved with a radius of curvature from 0.1 to 300 mm in a length range of equal to or less than 10 cm from the anastomosis part toward a downstream side to extend toward an upstream side of the second vessel from the anastomosis part.

3. The vascular corrective appliance according to claim 1 or 2, configured to form the shapes of the first vessel and the second vessel and retain the shapes in such a manner that the first vessel is rounded on a downstream side from the anastomosis part toward an upstream side of the second vessel from the anastomosis part, and that the rounding has a radius of curvature from 0.1 to 300 mm in a length range of equal to or less than 10 cm from the anastomosis part toward a downstream side.

4. The vascular corrective appliance according to claim 2 or 3, wherein the radius of curvature of the first vessel is from 10 to 50 mm.

5. The vascular corrective appliance according to any one of claims 1 to 4, wherein an angle formed between a blood flow direction of the first vessel and a blood flow direction of the second vessel at the anastomosis part is from 20 to 60 degrees.

6. The vascular corrective appliance according to any one of claims 1 to 5, configured to cover a predetermined range partially or entirely toward a downstream side of the first vessel from the anastomosis part, to cover a predetermined range partially or entirely toward an upstream side of the second vessel from the anastomosis part, and to cover a predetermined range partially or entirely toward a downstream side of the second vessel from the anastomosis part from the outer sides of the first vessel and the second vessel.

7. The vascular corrective appliance according to any one of claims 1 to 5, configured to cover a predetermined range partially or entirely toward a downstream side of the first vessel from the anastomosis part and to cover a predetermined range partially or entirely toward an upstream side of the second vessel from the anastomosis part from the outer sides of the first vessel and the second vessel.

8. The vascular corrective appliance according to any one of claims 1 to 5, configured to cover a predetermined range partially or entirely toward a downstream side of the first vessel from the anastomosis part and to cover a predetermined range partially or entirely toward a downstream side of the second vessel from the anastomosis part from the outer sides of the first vessel and the second vessel.

9. The vascular corrective appliance according to any one of claims 6 to 8, comprising two divisions dividable and combinable along a plane including the center line of the first vessel and the center line of the second vessel, wherein the vascular corrective appliance is configured to cover the first vessel and the second vessel by attaching and combining the divisions from opposite sides of each of the first vessel and the second vessel.

10. The vascular corrective appliance according to any one of claims 1 to 9, wherein each of the first vessel and the second vessel is an artery, a vein, or a vascular graft.

11. The vascular corrective appliance according to any one of claims 1 to 9, wherein the first vessel is a vein and the second vessel is an artery.

12. The vascular corrective appliance according to any one of claims 1 to 11, configured to be adjustable in terms of dimension and/or shape.

13. A supporting method for an anastomosis part that uses the vascular corrective appliance according to any one of claims 1 to 12 to support the first vessel and the second vessel anastomosed with each other.
